(19) Europäisches Patentamt
European Patent Office
Office européen des brevets

(11) Veröffentlichungsnummer: **0 150 290 B1**

(12) **EUROPÄISCHE PATENTSCHRIFT**

(45) Veröffentlichungstag der Patentschrift: **01.04.87**

(51) Int. Cl.⁴: **C 07 D 307/93** // A61K7/46

(21) Anmeldenummer: **84113721.9**

(22) Anmeldetag: **14.11.84**

(54) **Verfahren zur Herstellung von 13-Oxabicyclo [10.3.0]-pentadecan.**

(30) Priorität: **11.01.84 DE 3400690**

(43) Veröffentlichungstag der Anmeldung: **07.08.85 Patentblatt 85/32**

(45) Bekanntmachung des Hinweises auf die Patenterteilung: **01.04.87 Patentblatt 87/14**

(84) Benannte Vertragsstaaten: **AT BE CH DE FR GB IT LI NL**

(56) Entgegenhaltungen:
**DE - A - 2 810 107**
**DE - A - 3 040 994**
**DE - C - 2 136 496**

(73) Patentinhaber: **HÜLS AKTIENGESELLSCHAFT, Patentabteilung / PB 15 - Postfach 13 20, D-4370 Marl 1 (DE)**

(72) Erfinder: **Kaufhold, Manfred, Dr., Jasminweg 20, D-4370 Marl (DE)**

**EP 0 150 290 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein verbessertes Verfahren zur Herstellung von 13-Oxabicyclo[10.3.0]pentadecan.

13-Oxabicyclo[10.3.0]pentadecan ist ein wertvoller Ambra-Riechstoff mit sehr guter Haftfestigkeit. In der DE-OS 2 810 107 wird folgendes Herstellungsverfahren vorgeschlagen:

1. Umsetzung von Cyclododecanon I mit Bromessigester nach Reformatsky zum entsprechenden Hydroxyester II
2. Reaktion von II mit starken Säuren zum Lacton III
3. Reduktion von III zum Diol IV
4. Intramolekulare Wasserabspaltung in Gegenwart von p-Toluolsulfonsäure zum 13-Oxabicyclo-[10.3.0]pentadecan, das als Gemisch zweier Stereoisomerer vorliegt.

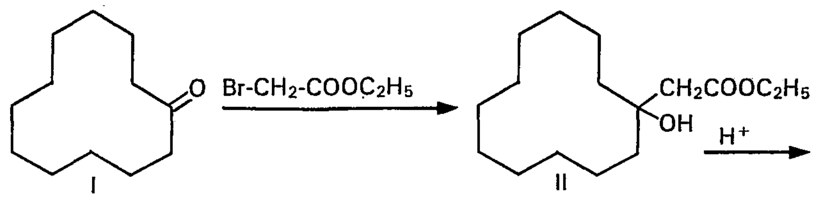


I II

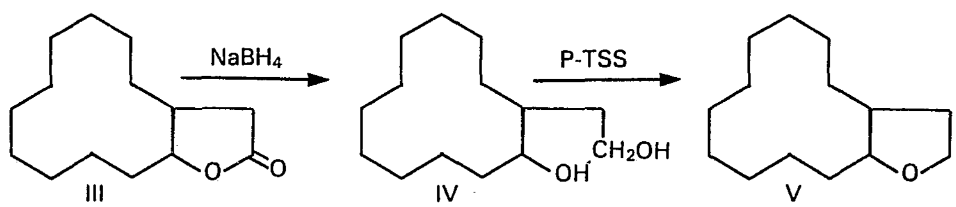


III IV V

Der bei diesem Verfahren eingesetzte Bromessigsäureester ist jedoch sehr toxisch und schleimhautreizend. Sein Einsatz erfordert daher besondere Sicherheitsmassnahmen. Zudem ist die Reformatzky-Synthese technisch schwer zu realisieren und ist nur von sehr gut geschultem Personal durchführbar.

Zur Vermeidung dieser Nachteile erfolgt die Herstellung von 13-Oxabicyclo[10.3.0]pentadecan nach dem Verfahren der DE-OS 3 040 994 über folgende Stufen:

1. Bromierung von Cyclododecanon in α-Stellung zur Carbonylgruppe zum Bromketon VI
2. Umsetzung des α-Bromcyclododecanon VI mit Natriummalonsäuredialkylester zum 2-(2-Oxocyclododec-1-yl)-malonsäuredialkylester VII
3. Verseifung unter Decarboxylierung von VII führt zur Ketocarbonsäure VIII
4. Veresterung von VIII mit einem niedrig siedenden Alkohol zum entsprechenden Ester IX
5. Reduktion des Ketoesters IX mit einem komplexen Metallhydrid wie $NaBH_4$ ergibt 2-(2-Hydroxyethyl)-cyclododecanol IV
6. Intramolekulare Wasserabspaltung aus IV zu V wie beim Verfahren der DE-OS 2 810 107.

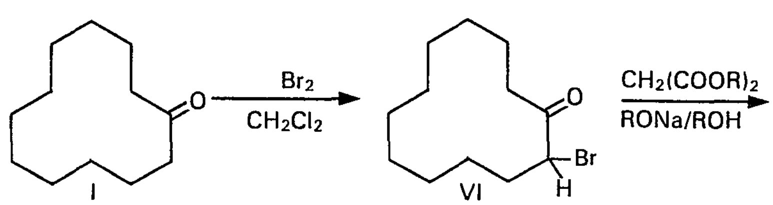


I VI

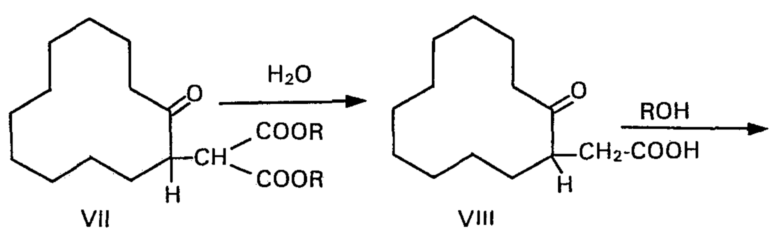


VII VIII

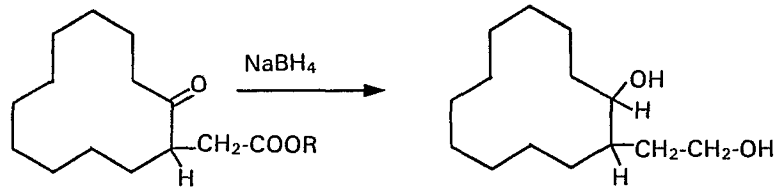


IX IV

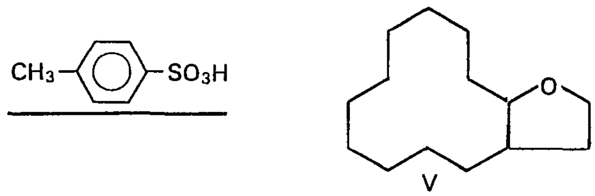

V

Auch dieses verbesserte Herstellungsverfahren weist noch einige Nachteile auf:

Brom und Natriummalonsäuredialkylester sind kostspielige Chemikalien und ihr Einsatz erfordert besondere technische Sicherheitsmassnahmen wie z.B. völligem Ausschluss von Feuchtigkeit bei der Umsetzung der Natriumverbindung. Zudem ist der Umgang mit dem toxischen Brom sowie die Umsetzung von VI zu VII, die nach den Angaben der DE-OS 3 040 994 «streng kontrollierte Reaktionsbedingungen» erfordert, nur von besonders gut ausgebildetem Personal durchzuführen.

Hieraus ergibt sich die Aufgabe, einen Syntheseweg ohne die genannten Nachteile zu finden, nach dem die Herstellung von 13-Oxabicyclo[10.3.0]pentadecan in einfacher Weise ohne besonderen technischen Aufwand mit leicht handhabbaren preiswerten Chemikalien von durchschnittlich ausgebildetem Personal möglich ist.

Diese Aufgabe wird erfindungsgemäss entsprechend den Angaben der Patentansprüche gelöst.

Die ersten drei Stufen dieses Syntheseweges sind neu. Nach diesem neuen Verfahren erhält man das Lacton in überraschend hohen Ausbeuten in technisch einfacher Weise.

In üblichen technischen Anlagen können beliebig grosse Mengen des wertvollen 13-Oxabicyclo-[10.3.0]pentadecan V ohne besondere Sicherheitsmassnahmen hergestellt werden.

Das neue Syntheseverfahren geht ebenfalls vom Cyclododecanon aus und wird wie folgt durchgeführt:

1. Umsetzung von Cyclododecanon mit Cyanessigsäure in Gegenwart eines üblichen Knoevenagel-Katalysators zum Cyclododecenylacetonitril X und XI, das als Isomerengemisch vorliegt.

2. Alkalische oder saure Verseifung des Nitrils X und XI und ggf. anschliessendes Ansäuern der bei alkalischer Verseifung erhaltenen Natriumsalze ergibt die Cyclododecenylessigsäure XII und die isomere Säure XIII.

3. Reaktion des Gemisches XII und XIII mit starken Säuren zum Lacton III.

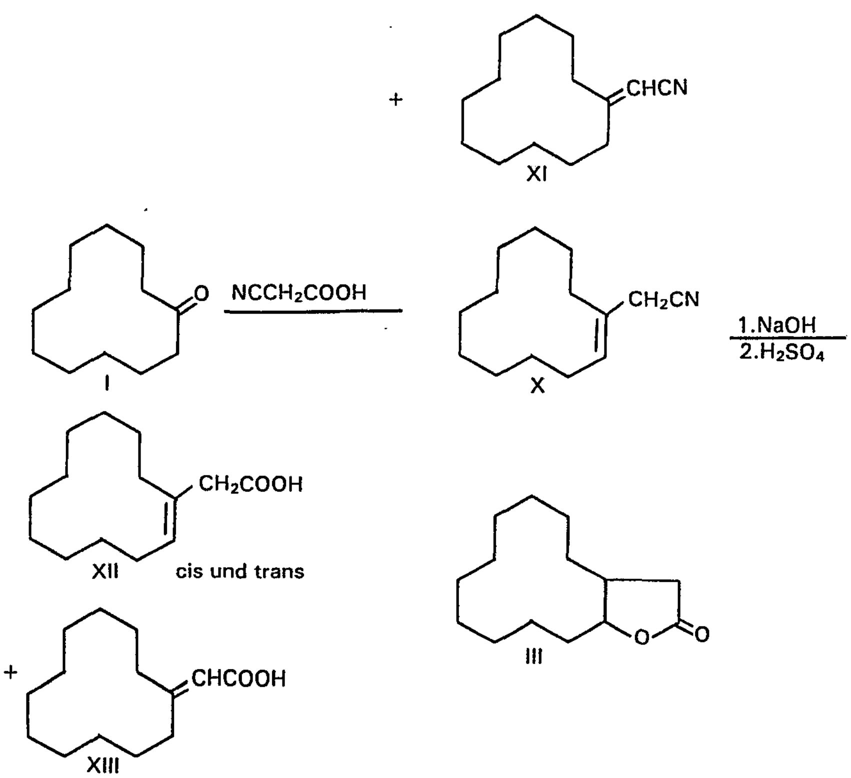


XI

I

X

XII cis und trans

XIII

III

4. und 5. Die Reduktion des Lactons III zum Diol IV wird katalytisch oder mit Hilfe von $NaBH_4$ durchgeführt. Man erhält das 2-(2-Hydroxyethyl)-cyclohexanol IV.

Die intramolekulare Wasserabspaltung des Diols zum 13-Oxabicyclo[10.3.0]pentadecan erfolgt nach dem Verfahren der DE-OS 2 810 107.

Durch Umsetzung von Cyclododecanon mit Cyanessigsäure in Gegenwart eines üblichen Knoevenagel-Katalysators, wie Ammoniumacetat, Piperidin, β-Alanin und andere Amine in Gegenwart von Eisessig erhält man das Cyclododecenylacetonitril-Gemisch (X und XI) in über 70%iger Ausbeute. Ammoniumacetat hat den Vorteil, dass es sehr preiswert ist, deshalb nicht zurückgewonnen werden muss und leicht von den Reaktionsprodukten abtrennbar ist.

In den Lehrbüchern der Organischen Chemie werden bei diesem Reaktionstyp — bekannt unter der Bezeichnung Knoevenagel-Reaktion — nur dann gute Ausbeuten genannt, wenn kurzkettige Ketone oder durch einen aromatischen Ring aktivierte Carbonylverbindungen eingesetzt werden. In der Reihe der Ketone, Aceton, Butanon und Cyclohexanon sinkt die Ausbeute von 90 über 80 auf 70%, d.h. mit wachsender Kettenlänge nimmt die Ausbeute stark ab (s. ORGANIKUM, 12. Aufl., VEB Deutscher Verlag der Wissenschaften, Berlin, 1973, Seite 508).

Es war also nicht zu erwarten, dass diese Reaktion bei Ringsystemen mit der im Vergleich zum Cyclohexanon doppelten C-Zahl wirtschaftlich interessante Ausbeuten liefert, insbesondere nicht, dass ein Keton mit 12 C-Atomen mit Cyanessigsäure und einem sehr schwach basischen Katalysator [im Gegensatz zu der Methode von Stephen A. DiBiase et al., Journal of Org. Chem. Vol. 44, Nr. 25 (1979), S. 4640] in über 70%iger Ausbeute das entsprechend substituierte Acetonitril ergibt.

Man setzt Cyclododecanon mit Cyanessigsäure im Molverhältnis von 5 : 1 bis 1 : 5, vorzugsweise von 1 : 2 bis 1 : 1 um in Gegenwart von 5 bis 1, vorzugsweise 3 bis 2 Gewichtsprozent eines üblichen Knoevenagel-Katalysators, insbesondere Ammoniumacetat, bezogen auf Cyclododecanon. Die Umsetzung erfolgt bei 100 bis 170°C, vorzugsweise 120 bis 140°C. Das erhaltene Reaktionsprodukt reinigt man destillativ bei 1 bis 50 mbar. Man erhält das Cyclododecenylacetonitril in einer Reinheit von über 99%.

Die Verseifung des so gewonnenen Cyclododecenylacetonitril-Gemisches X und XI erfolgt in üblicher Weise und kann mit Säuren oder Basen durchgeführt werden. Die alkalische Verseifung hat den Vorteil, dass die Aufarbeitung nicht durch ausgeschiedene Ammoniumsalze erschwert wird. Als Reaktionstemperatur ist der Bereich von 100 bis 200°C, vorzugsweise von 120 bis 180°C geeignet. Als Basen verwendet man Alkali- oder Erdalkalihydroxide, vorzugsweise wegen ihrer leichten Handhabung wässrige Natronlauge, und setzt sie im Molverhältnis Base : Nitrilgemisch von 10 : 1 bis 1 : 1, vorzugsweise 2 : 1 bis 1,05 : 1 ein.

Da während der alkalischen Verseifung Feststoffe ausfallen, ist es vorteilhaft, insbesondere wenn man bei Normaldruck und nicht so hohen Temperaturen (z.B. über 160°C) arbeiten will, ein Lösemittel oder Lösemittelgemische zuzusetzen.

Die Wahl des Lösemittels ist nicht kritisch. Bevorzugt setzt man stark polare, leicht zugängliche und preiswerte Lösemittel ein, wie z.B. Alkylglykole, Alkyldiglykole, Alkylpolyglykole, wobei als Alkylgruppe die Methyl-, Ethyl-, Propyl- oder Butylgruppe besonders geeignet ist, sowie Ethylenglykol, Diglykol, Triglykol, Polyglykol oder Gemische dieser Glykole bzw. Glykolderivate oder die entsprechenden Verbindungen, die vom Propylenglykol-1,2 stammen.

Der Fortgang der alkalischen Verseifung kann durch Messung des Ammoniakstromes festgestellt werden. Die erforderliche Zeit beträgt im allgemeinen ca. zwei bis sieben Stunden. Wenn die Ammoniakentwicklung beendet ist, säuert man mit einer Mineralsäure, vorzugsweise Schwefelsäure an und extrahiert mit einem inerten Lösemittel. Geeignete Lösemittel sind insbesondere aromatische Kohlenwasserstoffe wie Benzol, Toluol, Xylole und andere alkylische Benzole, vorzugsweise Toluol.

Es ist ein besonderer Vorteil des erfindungsgemässen Verfahrens, dass die Säure XII und XIII für die Weiterverarbeitung nicht zuvor gereinigt werden muss. Bei der destillativen Reinigung der Säure treten infolge Zersetzung erhebliche Verluste auf und auch die Reinigung durch Umkristallisieren wäre mit Verlusten verbunden.

Überraschenderweise wurde gefunden, dass die Cyclododecenylessigsäure, das Isomerengemisch XII und XIII, wie der Hydroxyester II mit starken Säuren wie Schwefelsäure in glatter Reaktion zum Lacton III reagiert. Die Umsetzung erfolgt im allgemeinen bei einer Temperatur von 40 bis 150°C, vorzugsweise 50 bis 100°C.

Diese Reaktion ist neu. Es war nicht zu erwarten, dass die Doppelbindung diese grosse Aktivität in der gewünschten Richtung zeigt. Da es sich bei XII um eine β,γ-ungesättigt Carbonsäure und bei XIII um eine substituierte Acrylsäure handelt, war unter den angewandten drastischen Bedingungen mit erheblicher Polymerisation zu rechnen.

Es ist weiterhin überraschend, dass man das so gewonnene Lacton III durch Destillation in sehr reiner Form erhält und es dann einen niedrigen Schwefelgehalt von kleiner als 10 ppm besitzt, während er vor der Destillation 50 ppm beträgt. Daher ist man bei dem nachfolgenden Syntheseschritt wegen eines möglichen Katalysatorgiftes nicht auf die aufwendige Reduktion mit einem relativ teuren Metallhydrid wie z.B. Natriumborhydrid wie beim Verfahren der DE-OS 2 810 107 angewiesen, sondern kann das Lacton III katalytisch hydrieren. Diese Reduktion kann zwar auch mit Natriumborhydrid erfolgen, bevorzugt hydriert man jedoch katalytisch, insbesondere mit Barium-aktivierten Kupferchromit-Katalysatoren bei Temperaturen von 150 bis 250°C und Wasserstoffdrücken von 200 bis 300 bar.

Der letzte Syntheseschritt ist identisch mit dem Verfahren der DE-OS 2 810 107 (Beispiel d). In dieser Schrift sind auch die besonderen Eigenschaften des 13-Oxabicyclo[10.3.0]pentadecans und seine Anwendung als Riechstoff beschrieben.

*Beispiel*

1.1 Synthese des Cyclododecenylacetonitrils

In einer Rührapparatur, die mit Thermometer, Rückflusskühler und Wasserauskreiser ausgestattet ist, werden folgende Produkte eingesetzt:

| | | |
|---|---|---|
| 434 g | (= 5 Mol) | Cyanessigsäure |
| 910 g | (= 5 Mol) | Cyclododecanon |
| 650 g | (= 5 Mol) | Toluol |
| 21 g | | Ammoniumacetat |

Bei 128 bis 130°C wird dieses Gemisch unter Rühren und Stickstoffabdeckung 30 Stunden lang am Rückfluss zum Sieden erhitzt und dabei das sich bildende Wasser, 80 g, ausgekreist. Hierbei entsteht ein Kohlendioxid-Strom.

Auswaage: 1667 g

Dieser Austrag wird an einer Brücke, die sich auf einer 10 cm langen mit 10 × 10 mm Glasraschigringen gefüllten Glaskolonne befindet, destilliert, wobei zunächst bei Normaldruck Toluol, 480 g, abdestilliert wird. In einem Siedebereich von 120 bis 180°C bei18 bis 20 mbar wird dann das Nitrilgemisch vom Rückstand, 89 g, abgestoppt.

Dieser Destillatanfall, 996 g, wird an einer 0,5 m langen mit Multifil-Füllkörpern gefüllten Glaskolonne fraktioniert. Bei 13 mbar werden 729 g Destillat in einem Siedebereich von 170 bis 179°C erhalten.

Die gaschromatographisch ermittelte Reinheit des Isomerengemisches liegt bei 99,1%; die Gehalte der einzelnen Komponenten waren:

1. Komponente 63,2%
2. Komponente 32,0%
3. Komponente 3,9%

Die Komponenten 1 und 2 sind der Struktur X zuzuordnen und sind trans-cis Isomere. Die 3. Komponente hat wahrscheinlich die Struktur XI.

Die Ausbeute an X und XI errechnet sich zu 71,1%, bezogen auf den Einsatz.

1.2 Nitrilverseifung

In einer Rührapparatur, die mit Thermometer, Rückflusskühler und Tropftrichter ausgestattet ist und durch die ein leichter Stickstoffstrom geleitet wird, der nach dem Rückflusskühler durch eine mit 800 g Wasser gefüllte Waschvorlage geht, werden folgende Produkte eingesetzt:

| | | |
|---|---|---|
| 410 g | (= 2 Mol) | Cyclododecenylacetonitrilgemisch (99,6%ig) |
| 40 g | | Ethylenglykol |
| 0,4 g | | tert.-Butyl-brenzcatechin (als Stabilisator) |

Das Gemisch wird unter Rühren auf 154°C erwärmt. Wie in der folgenden Übersichtstabelle aufgeführt, wird 50%ige Natronlauge, insgesamt 240 g (= 3 Mol) = 160 ml und einmal 20 g und einmal 10 g Wasser zugegeben. Die Tabelle zeigt auch den Ammoniakgehalt im Waschwasser und den Temperaturverlauf.

| Zeit (h) | Temperatur (°C) | Natronlauge-zugabe (ml) | Wasser-zugabe (ml) | Ammoniak-gehalt (%) |
|---|---|---|---|---|
| Beginn | 154 | — | — | — |
| 0,5 | 134 | 30 | — | nicht gemessen |
| 1,0 | 134 | 70 | — | » |
| 1,5 | 130 | 160 | 20 | 2,77 |
| 2,5 | 128 | — | — | 3,3 |
| 3,5 | 128 | — | 10 | 3,42 |
| 4,5 | 125 | — | — | 3,48 |
| 5,5 | 125 | | | 3,50 |
| 6,5 | 125 | | | 3,67 |

Dann kühlt man auf ca. 100°C ab und gibt unter Rühren 400 g Toluol und 400 g Wasser zu. Anschliessend wird auf Raumtemperatur abgekühlt und mit 738 g 20%iger Schwefelsäure bis auf einen pH-Wert von3,0 angesäuert.

Die Phasentrennung ergibt:

1381 g wässrige und 825 g Öl-Phase.

Die wässrige Phase wird mit 50 g Toluol extrahiert. Der Kohlenstoffgehalt dieses Abwassers liegt dann bei 1,97%.

Die Ölphasen werden zusammengegeben und das Toluol an einer Brücke, die sich auf einer 10 cm langen leeren Kolonne befindet, zunächst bei 200 mbar, dann bei 100 und schliesslich bei 20 mbar bei Sumpftemperaturen von jeweils maximal 100°C abdestilliert.

Destillatmenge: 437 g
Rückstandsmenge: 431 g

Nach Säurezahl dieses Rückstandes (SZ = 215,1) ist die Rohsäure 85,9%ig. Auf diesen Gehalt und den Einsatz bezogen errechnet sich die Ausbeute von 82,6%.

1.3 Lactonbildung

In einer Rührapparatur wird unter Stickstoffabdeckung vorgelegt:

2727 g (= 22,3 Mol) Schwefelsäure (80%ig).

Die Schwefelsäure wird auf 60°C erwärmt und unter kräftigem Rühren wird die rohe Cyclododecenylessigsäure, 418 g (= 1,6 Mol) 85,9%ig, innerhalb von 30 Minuten zugetropft. Danach wird noch eine Stunde bei 60°C gerührt.

In einer zweiten Rührapparatur werden 2 l auf 10°C gekühltes Wasser vorgelegt und unter Kühlen wird das Reaktionsgemisch innerhalb von 2,5 Stunden so zugegeben, dass die Temperatur bei ca. 10°C gehalten werden kann.

Dann wird zuerst mit 600 g Toluol und danach mit 300 g Toluol extrahiert. Abwassermenge: 4741 g mit Gehalt an Schwefelsäure von 45,8% und an Kohlenstoff von 0,79%.

Die Ölphasen aus der Extraktion, 991 und 298 g, werden zusammengegeben und mit 150 g einer 10%igen Sodalösung neutral gewaschen. Abwassermenge: 183 g.

Die Ölphase (1258 g) wird an einer 0,5 m langen mit Multifil-Füllkörpern gefüllten Glaskolonne in folgender Weise destilliert:

| Fr. Nr. | Temperaturen (°C) Sumpf | Temperaturen (°C) Kopf | Druck (mbar) | Gewicht (g) | Verhältnis Rücklauf zur Abnahme |
|---|---|---|---|---|---|
| 1 | 116-170 | 87-110 | 1013 | 805 | 3 : 1 |
| | 96-198 | 37- 46 | 13 | | 10 : 1 |
| 2 | 214-222 | 131-194 | 13 | 43 | 5 : 1 |
| 3 | 222 | 194-202 | 13 | 9 | 5 : 1 |
| 4 | 222-252 | 202-209 | 13 | 270 | 30 : 1 |
| | | | | | 3 : 1 |
| | Rückstand | | | 13 | |
| | Kühlfalle | | | 77 | |
| | | | | 1217 | |

Das Kühlfallenprodukt wird zur Fraktion 1 gegeben und das Gemisch analysiert; es ist reines Toluol.

Die Fraktion 2 besteht zu 97,2% aus Cyclododecanon.

Die Fraktionen 3 und 4 sind nach GC-Analyse wie folgt zusammengesetzt:

| | Fr.Nr. 3 | Fr.Nr. 4 |
|---|---|---|
| Vorlauf | 2,7 | 0,1 |
| Cyclododecanon | 75,0 | 0,2 |
| Zwischenlauf | 1,7 | |
| Komponente 1 Kp ca. 365°C | 14,5 | 62,9 |
| Komponente 2 Kp ca. 365°C | 1,7 | 31,1 |
| Komponente 3 Kp ca. 365°C | 0,3 | 5,2 |

Die Komponenten 1, 2 und 3 betreffen das gewünschte Lacton.

Die Ausbeute an Lacton errechnet sich aus diesen Zahlen zu 75,3%, bezogen auf eingesetzte Rohsäure.

Der Schwefelgehalt des Lactons lag vor der Destillation bei 50 ppm und nach der Destillation im Hauptlauf bei nur 6 ppm.

1.4.1 Die Reduktion des Lactons wird mit Natriumborhydrid in der gleichen Weise durchgeführt wie sie in der DE-OS 2 810 107, Beispiel C, beschrieben ist:

«Das erhaltene rohe Lacton (204 g) wurde in 3600 ml Isopropanol gelöst. Unter Rühren wurden 45,6 g (1,2 Mol) Natriumborhydrid zugesetzt und das Gemisch 3 Stunden unter Rühren am Rückfluss erhitzt. Nach beendeter Reaktion wurde das Gemisch in die zweifache Menge Wasser eingegossen, das Gemisch mehrfach mit Äther extrahiert, die Extrakte getrocknet, das Lösungsmittel entfernt und das Rohprodukt im Ölpumpenvakuum destilliert.

Das erhaltene Diol stellt eine farblose viskose Flüssigkeit dar mit einem $Kp_{0,6}$ von 172-175°C».

1.4.2 Die katalytische Hydrierung des Lactons erfolgt nach Lösen in n-Butanol im Verhältnis 1 : 1 bei 200°C und 300 bar Wasserstoffdruck über einen mit Barium aktivierten Kupferchromitkontakt der Zusammensetzung:

ca. 33% CuO, ca. 38% $CrO_3$, ca. 10% BaO, Rest $SiO_2$ (1300 ml).

Als Reaktor wird ein 1,5-l-Hochdruckreaktor eingesetzt und 250 ml/h der Lösung kontinuierlich zudosiert.

Die Katalysatorbettbelastung beträgt 0,096 l Lacton/l Kontakt · h.

Der Hydrieraustrag hat eine Säurezahl von 0,02 und eine Verseifungszahl von 0,3, d.h. der Umsatz an Lacton liegt über 99%. Nach dem Abdestillieren des Butanols wird der Destillationsrückstand direkt weiterverarbeitet.

1.5 Die intramolekulare Wasserabspaltung erfolgt in der gleichen Weise wie sie in der DE-OS 2 810 107, Beispiel d, beschrieben ist:

«Das erhaltene Diol (208 g) wurde in 640 ml Toluol gelöst, 17,2 g p-Toluolsulfonsäure zugesetzt und die Lösung 2 Stunden am Wasserabscheider erhitzt. Nach Beendigung der Reaktion wurde mit 2n-Sodalösung, danach mit Wasser, neutral gewaschen und die Lösung über Natriumsulfat getrocknet. Nach Entfernen des Lösungsmittels wurde im Ölpumpenvakuum destilliert».

Man erhält das 13-Oxabicyclo[10.3.0]pentadecan als Isomerengemisch (Konzentrationen der Isomere: 75,7 und 23,2%, in 98,9%iger Reinheit mit guter Geruchsqualität.

**Patentansprüche**

1. Verfahren zur Herstellung von 13-Oxabicyclo-[10.3.0]pentadecan aus Cyclododecanon, dadurch gekennzeichnet, dass man

a) Cyclododecanon mit Cyanessigsäure im Molverhältnis von 5 : 1 bis 1 : 5 bei Temperaturen von 100 bis 170°C in Gegenwart von 5 bis 1 Gewichtsprozent eines üblichen Knoevenagel-Katalysators, bezogen auf Cyclododecanon, umsetzt,

b) das so gewonnene Nitril alkalisch oder sauer bei einer Temperatur von 100 bis 200°C verseift, bei der alkalischen Verseifung mit einer Mineralsäure ansäuert, die erhaltene Cyclododecenylessigsäure mit einem inerten Lösemittel extrahiert und die Säure nach dem Abdestillieren des Lösemittels gewinnt,

c) die Cyclododecenylessigsäure mit starken Säuren zum Lacton cyclisiert,

d) das Lacton katalytisch oder mit Natriumborhydrid zum entsprechenden Diol reduziert und

e) das Diol in bekannter Weise unter sauer katalysierter intramolekularer Wasserabspaltung zum 13-Oxabicyclo[10.3.0]pentadecan umsetzt.

2. Verfahren nach Anspruch 1, dadurch gekennzeichnet, dass man als Knoevenagel-Katalysator 3 bis 2 Gewichtsprozent Ammoniumacetat einsetzt.

3. Verfahren nach Anspruch 1 und 2, dadurch gekennzeichnet, dass man die Stufe a) bei 120 bis 140°C durchführt.

4. Verfahren nach Anspruch 1, dadurch gekennzeichnet, dass man die Nitrilverseifung alkalisch in Gegenwart eines stark polaren Lösemittels oder Lösemittelgemisches unter Zugabe von Wasser während der Reaktion durchführt.

5. Verfahren nach Anspruch 1 und 4, dadurch gekennzeichnet, dass man bei der alkalischen Nitrilverseifung ein Molverhältnis Base : Nitrilgemisch von 10 : 1 bis 1 : 1, vorzugsweise 2 : 1 bis 1,05 : 1 einsetzt.

6. Verfahren nach Anspruch 1, dadurch gekennzeichnet, dass man in Stufe c) als starke Säure Schwefelsäure einsetzt.

7. Verfahren nach Anspruch 1, dadurch gekennzeichnet, dass man zur Lactonbildung die rohe Cyclododecenylessigsäure einsetzt und das erhaltene Lacton destillativ reinigt.

7. Verfahren nach Anspruch 1, dadurch gekennzeichnet, dass man die katalytische Reduktion des Lactons mit Barium aktivierten Kupferchromit-Katalysatoren bei 150 bis 250°C durchführt.

**Claims**

1. A process for the production of 13-oxabicyclo-[10.3.0]-pentadecane from cyclododecanone, characterised in that

a) cyclododecanone is reacted with cyanoacetic acid in a mole ratio of from 5 : 1 to 1 : 5 at a temperature of 100 to 170°C in the presence of 5% to 1% by weight, based on cyclododecanone, of a conventional Knoevenagel catalyst;

b) the resulting nitrile is saponified at a temperature of 100 to 200°C under alkaline or acid conditions, and in the case of alkaline saponification the product is acidified with a mineral acid, the cyclododecenylacetic acid obtained is extracted with an inert solvent and the acid is recovered after distilling off the solvent;

c) the cyclododecenylacetic acid is cyclised to the lactone with the aid of a strong acid;

d) the lactone is reduced to the corresponding diol catalytically or with sodium borohydride; and

e) the diol is converted in a known manner by acid-catalysed intramolecular dehydration to form 13-oxabicyclo[10.3.0]-pentadecane.

2. A process according to claim 1, characterised in that 3 to 2% by weight of ammonium acetate is used as Knoevenagel catalyst.

3. A process according to claim 1 or 2, characterised in that step a) is carried out at 120 to 140°C.

4. A process according to claim 1, characterised in that the saponification of the nitrile is carried out under alkaline conditions in the presence of a strongly polar solvent or solvent mixture with the addition of water during the reaction.

5. A process according to claim 1 or 4, characterised in that a mole ratio of base : nitrile mixture of from 10 : 1 to 1 : 1, preferably from 2 : 1 to 1.05 : 1, is introduced in carrying out the alkaline saponification of the nitrile.

6. A process according to claim 1, characterised in that sulphuric acid is introduced as strong acid in step c).

7. A process according to claim 1, characterised in that the crude cyclododecenylacetic acid is introduced for the formation of the lactone, and the lactone obtained is purified by distillation.

8. A process according to claim 1, characterised in that the catalytic reduction of the lactone is carried out with a barium-activated copper chromite catalyst at 150 to 250°C.

**Revendications**

1. Procédé de préparation de 13-oxabicyclo [10.3.0] pentadécane en partant de cyclododécanone, caractérisé par le fait

a) que l'on fait réagir la cyclododécanone sur l'acide de cyanacétique dans un rapport molaire de 5 : 1 à 1 : 5, à des températures de 100 à 170°C, en présence de 5 à 1% d'un catalyseur Knoevenagel usuel, relativement à la cyclododécane,

b) que l'on saponifie le nitrile ainsi obtenu en milieu alcalin ou acide à une température de 100 à 200°C, que dans le cas de la saponification alcaline on acidifie par un acide minéral, que l'on extrait l'acide cyclododécénylacétique obtenu par un solvant inerte et que l'on obtient l'acide après avoir chassé le solvant par distillation,

c) que l'on cyclise l'acide cyclododécénylacétique avec des acides forts pour obtenir la lactone,

d) que l'on réduit la lactone, catalytiquement ou par l'hydruroborate de sodium, en diol correspondant et

e) que l'on convertit le diol de façon connue en 13-oxabicyclo [10.3.0] pentadécane avec déshydratation intramoléculaire catalysée par un acide.

2. Procédé selon la revendication 1, caractérisé

par le fait que comme catalyseur Knoevenagel, on utilise de 3 à 2% en poids d'acétate d'ammonium.

3. Procédé selon la revendication 1 et 2, caractérisé par le fait que l'on conduit l'étape a) à une température de 120 à 140°C.

4. Procédé selon la revendication 1, caractérisé par le fait que l'on conduit la saponification alcaline du nitrile en présence d'un solvant fortement polaire ou d'un mélange de solvants fortement polaires, avec addition d'eau pendant la réaction.

5. Procédé selon la revendication 1 et 4, caractérisé par le fait que lors de la saponification alcaline du nitrile, on utilise un rapport molaire entre base et mélange de nitrile de 10 : 1 à 1 : 1, de préférence de 2 : 1 à 1,05 : 1.

6. Procédé selon la revendication 1, caractérisé par le fait qu'à l'étape c), on utilise comme acide fort l'acide sulfurique.

7. Procédé selon la revendication 1,caractérisé par le fait que pour la formation de la lactone, on utilise l'acide cyclododécénylacétique brut et que l'on purifie par distillation la lactone obtenue.

8. Procédé selon la revendication 1, caractérisé par le fait que l'on conduit la réduction catalytique de la lactone avec des catalyseurs au chromite de cuivre activés par le baryum, à une température de 150 à 250°C.